# EUROPEAN PATENT APPLICATION

(11) **EP 4 497 377 A1**
(43) Date of publication of application: **29.01.2025**
(21) Application number: 23306290.0
(22) Date of filing: 25.07.2023
(51) Int. Cl.: A61B 5/0533, A61B 5/00, A61N 5/06

(54) **SYSTEM FOR EVALUATING A LIGHT-INDUCED DISCOMFORT AND ASSOCIATED METHOD**

(71) Applicant: Essilor International, 94220 Charenton-Le-Pont (FR); SORBONNE UNIVERSITE, 75006 Paris (FR)
(72) Inventor: LASSALLE, Astrid, 94100 SAINT MAUR DES FOSSES (FR); EHRISMANN, Camille, 49130 LES PONTS DE CÉ (FR); DUBAIL, Marie, 94410 SAINT MAURICE (FR)
(74) Representative: Plasseraud IP

(57) **Abstract**

A system (3) for evaluating a light-induced discomfort of a user, the system (3) comprising a sensor (9) for measuring an electrical conductance of a skin surface of the user, the system (3) being configured for:
- obtaining through the sensor (9) at least one measurement of the electrical conductance of the skin surface of the user,
- obtaining at least one value of at least one parameter representative of the at least one measurement of the electrical conductance, and
- comparing the at least one value of the at least one parameter to at least one reference value and detecting a light-induced discomfort of the user based on the comparison.

## Description

### Field of the invention

The invention relates to the field of evaluation and reduction of light-induced discomfort.

More specifically, the invention relates to a system for evaluating light-induced discomfort in a user.

The invention also relates to a computer-implemented method implemented by a system.

### Background of the invention

The effect of ambient light on stress and well-being is a previously observed and very relevant matter in the field of optical devices and other light-related applications such as luminotherapy.

Light exposure, both during a short duration and through the day, impacts wellbeing and effective management of this impact may be a key parameter to improve it.

Exposure to varying amounts of light may also have an impact on alertness and emotional state, with studies showing variations in response time and response accuracy to simple questions, based on light exposure of the subjects.

There is thus interest in identifying optimal light conditions to improve alertness and/or comfort, reduce stress, and issue recommendations for adjusting light exposure using tinted lenses, for example.

However, these effects are difficult to analyse precisely and in a quantitative way, as most studies so far rely on qualitative observations to determine a comfort level of the subjects being tested. Feedback on discomfort can be obtained through questionnaires or specific devices requiring user input to detect discomfort.

However, internal studies made by the applicant showed that subjective assessment of light sensitivity based on illuminance levels lacks repeatability and suffers from differences between ages, as older people for example tend to declare themselves sensitive more rarely. In addition, questionnaires and the guidelines for providing user input can be hard to understand for the tested subjects, since light discomfort does not have a commonly accepted definition.

Besides, the perception of light discomfort varies with time, which can be hard to take into account in relation to a punctual illuminance measurement.

Furthermore, integrating this type of observation in an automated device or procedure would require active user participation and thus greatly reduce the benefits of automation.

There is thus a need for a system for evaluating a light-induced discomfort of a user in a precise and quantitative manner, without relying in direct input from the user and issue recommendations and/or commands based on the detected discomfort.

### Summary of the invention

To this end, the invention relates to a system for evaluating a light-induced discomfort of a user, the system comprising a sensor for measuring an electrical conductance of a skin surface of the user, the system being configured for:
- obtaining through the sensor at least one measurement of the electrical conductance of the skin surface of the user,
- obtaining at least one value of at least one parameter representative of the at least one measurement of the electrical conductance, and
- comparing the at least one value of the at least one parameter to at least one reference value and detecting a light-induced discomfort of the user based on the comparison.

Such a system allows for a quantitative measurement of a physiological parameter leading to a detection of an eventual light-induced discomfort of the wearer.

According to an embodiment, the at least one parameter may comprise a frequency of spikes of the measured electrical conductance, an amplitude of spikes of the measured electrical conductance, and/or a delay between spikes of the measured electrical conductance.

Such a parameter has been shown to be correlated with discomfort experienced by the user and can be easily determined by numerical treatment by a control module for easy implementation in a control method.

According to another embodiment, the system may comprise a notification module configured to issue notifications to the user, through an interface and/or through an external electronic device, the system being also configured for:
- issuing a notification of a detected light-induced discomfort of the user and, optionally, a suggestion to adjust or replace an optical element worn by the user.

Such a feature allows to warn the user of a detected light-induced discomfort so that a correction can be implemented.

According to another embodiment, the system may comprise a communication module configured to communicate with at least one controllable optical element presenting a variable optical function worn by the user, the system being also configured for:
- issuing a command to the controllable optical element to modify the variable optical function of the controllable optical element to reduce the detected light-induced discomfort of the user.

Such a features allows an automated response to the perceived discomfort of the user by varying the optical function of the optical element to reduce said discomfort.

According to another embodiment, the controllable optical element may be an dimming element, the command to modify the optical function comprising adjusting a transmission value of the dimming element and/or a polarisation value of the dimming element and/or a transition speed between at least two states of the dimming element.

Such a feature allows to automatically adapt the transition of a dimming element to reduce a perceived stress of the user without requiring user input.

According to another embodiment, the system may comprise a communication module configured to communicate with a luminotherapy device in use by the user, the system being also configured for:
- issuing a command to the luminotherapy device of adjusting settings of the luminotherapy device to reduce the detected light-induced discomfort of the user.

Such a feature allows an automatic control of a luminotherapy device to adjust the parameters, such as colour and intensity, to cause minimal discomfort to the user, without requiring user input.

According to another embodiment, the sensor may be arranged in a frame of a head-mounted device or arranged in an external device worn by the user in contact with the skin surface of the user.

Such a feature allows continuous measurements of the electrical conductance during use of the system, in a portable and convenient manner.

According to another embodiment, the system may further comprise at least one sensor for determining an amount of an ambient light, the system being also configured for:
- comparing the amount of the ambient light with a predetermined ambient light threshold,
- detecting the light-induced discomfort also based on the comparison of the amount of the ambient light with a predetermined ambient light threshold.

Such a feature allows an automated detection of an excessive light amount that may be the cause for a perceived discomfort, thus reducing the risk of wrongly detecting a discomfort caused by another source.

According to another embodiment, the system may further comprise at least one position and/or motion sensor, the system being also configured for:
- obtaining values of the at least one activity parameter representative of a position and/or a motion of the user through the at least one position and/or motion sensor,
- determining an activity-induced stress level based on the values of the at least one activity parameter, and
- adjusting the reference values based on the determined activity-induced stress level.

Such a feature allows taking into account the nature and level of activity being performed by the user to prevent false positives in the detection of a light-induced stress.

According to another embodiment, the system may further comprise at least one physiological sensor configured for obtaining values of at least one physiological parameter of the user, the system being also configured for:
- obtaining values of the at least one physiological parameter of the user through the at least one physiological sensor,
- determining a physiological stress level based on the values of the at least one physiological parameter, and
- adjusting the reference values based on the determined physiological stress level.

Such a feature allows the use of additional parameters in the determination of the stress level of the user, to improve the accuracy of detection of stress. This physiological parameter may be for example heart rate, blood pressure, skin temperature, of even electric activity in the brain region.

According to another embodiment, the reference values may be determined specifically for the user in a preliminary calibration process.

Such a feature allows to take into account personal variation in sensitivity to light-induced stress and response in variation of skin conductance.

According to another embodiment, the system may also comprise a calculation module comprising at least one processor and at least one memory.

The invention also relates to a head-mounted device comprising a frame, at least one optical element and a system for evaluating a light-induced discomfort of a user, the system comprising a sensor for measuring an electrical conductance of a skin surface of the user, the system being configured for:
- obtaining through the sensor measurements of the electrical conductance of the skin surface of the user,
- obtaining at least one value of at least one parameter representative of the measured electrical conductance, and
- comparing the at least one value of the at least one parameter to at least one reference value and detecting a light-induced discomfort of the user based on the comparison.

The invention further relates to eyeglasses comprising a frame, at least one optical element and a system for evaluating a light-induced discomfort of a user, the system comprising a sensor for measuring an electrical conductance of a skin surface of the user, the system being configured for:
- obtaining through the sensor measurements of the electrical conductance of the skin surface of the user,
- obtaining at least one value of at least one parameter representative of the measured electrical conductance, and
- comparing the at least one value of the at least one parameter to at least one reference value and detecting a light-induced discomfort of the user based on the comparison.

The invention also relates to a computer-implemented evaluation method for evaluating a light-induced discomfort of a user, the evaluation method comprising:
- obtaining through the sensor measurements of the electrical conductance of the skin surface of the user,
- obtaining at least one value of at least one parameter representative of the measured electrical conductance, and
- comparing the at least one value of the at least one parameter to at least one reference value and detecting a light-induced discomfort of the user based on the comparison.

### Brief description of the drawings

Figure 1 is a schematic representation of a head mounted device comprising a system according to the invention,
Figure 2 is a graphical representation of measured skin conductance of a user of the system of figure 1,
Figure 3 is a schematic representation of a system according to another embodiment of the invention,
Figure 4 is a schematic representation of a system according to a third embodiment of the invention, and,
Figure 5 is a representation of an evaluation method according to the invention.

### Detailed description of embodiments

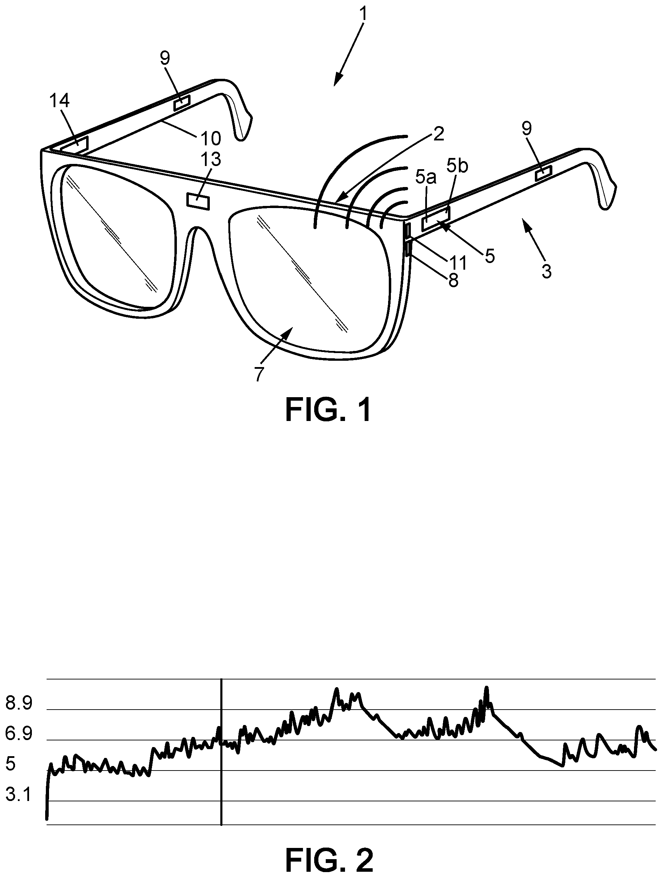

A first implementation of the invention is described below, in reference to figure 1.

In this implementation, a system 3 for evaluating light-induced discomfort according to the invention is mounted on a head-mounted device 1 of the type commonly designated as smart glasses, shown on figure 1.

Such a head-mounted device 1 typically comprises a rigid frame 2 comprising a single part or several articulated parts, arranged for being put on the face of a wearer by resting on at least one facial feature such as the nose and/or ears.

In this implementation, the entire system 3 is mounted in the device 1, and comprises an electronic calculation module 5, at least one optical element 7, as well as at least one sensor 9 configured for measuring values of an electric conductance of a skin surface of the wearer.

The calculation module 5 comprises an electronic circuit comprising at least one processor 5a, for executing programs, and a memory 5b for storing data and instructions for the execution of said programs.

In the example described, the optical element 7 is an optical lens mounted on the head-mounted device 1.

The optical element 7 may be an ophthalmic lens providing an ophthalmic correction to the user. Alternatively, the optical element 7 may be a plano lens, providing no ophthalmic correction.

An ophthalmic lens or a plano lens may also be conformed to modify an intensity of the light traversing the lens, such as a colored absorbing lens, or sunglasses with a gradient of transmission.

Alternatively, the optical element 7 may have at least one variable optical function, such as a variable transmission lens.

The variable optical function may vary automatically, such as when exposed to incident light, or be piloted, such as for under a control of the calculation module 5.

For example, as shown on figure 1, the optical element 7 may be a dimming element, for example an electrochromic lens comprising an electrochromic element and a control module 8.

The dimming element may transition between at least two states, preferably in a controlled manner, the variable optical function taking different values in the different states of the dimming element.

The variable optical function may be a variable tint level and/or a variable polarization of the dimming element.

The control module 8 is configured to modify a current value of the variable optical function under control from the calculation module 5, for example by applying a control tension on the dimming element derived from a command received from the calculation module 5.

Furthermore, the system 3 may be configured to adjust both a desired value of the optical function, such as a desired tint level, as well as a transition speed from the current value to the desired value of the optical function and between two different states of the dimming element.

The sensor 9 is for example a contact sensor that must be placed in contact with the skin region to allow accurate measurements. The sensor 9 is thus mounted on the smart glasses to be in contact with the skin region, when the glasses are worn, such as, in the example shown on figure 1, in branches 10 of the frame, to be in contact with the temples of the user, or between the lenses to contact the nose bridge.

Alternatively, the sensor 9 may be mounted on an external device worn in contact with the user's skin, such as a bracelet or watch, and anklet, a belt, a headband, etc... In this case, the sensor 9 is in communication with the control module through a direction connection or a wireless connection.

The system 3 is configured to obtain through the sensor 9 at least one measurement of the electrical conductance of the skin surface of the user and derive therefrom at least one value of at least one parameter representative of the measured electrical conductance.

Figure 2 is a graphical representation of an example recording of the variations of the electrical conductance EDA of a skin region of a user, expressed in microSiemens (µS) against time, showing a number of spikes with varying frequency and intensity.

Advantageously, the at least one parameter comprises a frequency of the spikes of the measured electrical conductance, an amplitude of the spikes of the measured electrical conductance, and/or a delay between spikes of the measured electrical conductance.

Both the frequency and intensity of the spikes have been shown to be correlated with elevated stress levels in the user.

For example, the frequency of the spikes of the measured electrical conductance may be between 0,045Hz and 0,25Hz during regular activities, between 0.25Hz and 0.28Hz during moderate activities and put to 0.37 during vigorous intensity activities.

The system 3 is also configured for selecting at least one predetermined reference value for the at least one parameter, from preregistered reference values stored in the memory 5b.

The selected reference values are advantageously selected based on the specific preferences of the user and/or based on environmental and contextual features.

The system 3 is also configured for comparing the at least one value of the at least one parameter to the at least one reference value and detecting a light-induced discomfort of the user based on the comparison.

For example, the reference value may be a threshold for the spikes intensity and/or the spikes frequency, that, when crossed, triggers a detection of a perceived stress in the user.

The system 3 may be configured for determining the reference values specifically for the user in a preliminary calibration process.

The reference values are stored on the memory and are advantageously specific of the user and loaded when the device 1 is turned on and the identity of the user is confirmed.

The head-mounted device 1 may also comprise a communication device 11, or communication interface, for enabling communication between the head-mounted device and other electronic devices such as a computer, a smartphone, or other elements in a personal area network of the user.

The communication device 11 may function through a direct wire connection or through a wireless connection protocol, such as "Wi-Fi" or "Bluetooth", for example.

The system 3 may comprise a notification module 6 configured to issue notifications to the user, through an interface and/or through an external electronic device, using the communication device 11.

The interface may comprise a visual interface, such as a screen or a light-emitting signal device. Alternatively, the interface may include an audio interface to emit an audio signal, or a vibratory interface to emit an acoustic signal to the user.

The system 3 may be configured to issue at least one notification to the user is case of a perceived light-induced stress or discomfort of the user, and to submit suggestions to address the perceived stress. For example, the system 3 may suggest adjustments of optical articles in use by the user, such as the use of sunglasses to reduce discomfort caused by excessive ambient light.

The notification module 6 may also be configured to issue notification to an eyewear practician to provide data for a visual diagnostic of the user and to adjust optical devices used.

The system 3 may also be configured to control the least one controllable optical element with a a variable optical function worn by the user, based on the detected light-induced discomfort.

The system 3 may be configured to control the optical element through a direct connection of through the communication device 11.

The system 3 is then also configured to issue a command to the controllable optical element to modify the variable optical function to reduce the detected light-induced discomfort of the user.

For example, in case of a perceived discomfort caused by a high level of ambient light, the system 3 may reduce the transmission value of the lenses worn by the user.

Alternatively, in case of a too fast or too slow transition of the transmission value, a resulting perceived stress may lead to a correction in the transition speed to reduce discomfort.

This feature allows an adaptation of the dynamics of transition when the user is exposed to abrupt and/or repetitive transitions between a bright and a dark environment or vice-versa.

The system 3 may also comprise a light sensor 13, configured to measure an amount of ambient light around the user.

The light sensor 13 may be mounted on the head-mounted device 1 or on an external device, in communication with the calculation module 3 through the communication device 11. In the example shown on figure 1, the light sensor 13 is embedded on the frame 2 of the head-mounted device 1.

The system 3 is in case configured to take into account the measured ambient light level when determining an eventual light-induced discomfort and when determining suggestions to issue to the user. For example, a high level of ambient light is more conductive to light-induced discomfort, while a low level of ambient light indicates that other sources of stress may be responsible of a perceived stress.

The system 3 may further comprise at least one position and/or motion sensor 14, either mounted on the head-mounted device 1 as shown on figure 1, or on an external device.

The at least one motion sensor 14 may comprise an inertial motion sensor (also known as an IMU, for Inertial Motor Unit), which measures linear acceleration (i.e. acceleration in a straight line), a gyroscopic sensor, which measures angular movements and positions, a magnetic sensor, for measuring orientations relative to a reference direction, as well as any sensor that measures information that can be related to movement or position.

The at least one sensor 14 may also include at least one front-facing camera mounted on the head-mounted device 1, configured to acquire images of the environment in front of the user and associated with appropriate image treatment algorithm to deduce form the obtained image a level of motion or activity by the user.

The at least one motion or position sensor 14 may also comprise a GPS unit, configured to determine a location of the user.

The system 3 is then configured to obtain values of at least one activity parameter representative of a position and/or a motion of the user through the at least one position and/or motion sensor 14 and determine an activity-induced stress level based on the values of the at least one activity parameter.

The sensor 3 is then configured to adjust the selected reference values based on the determined activity-induced stress level. For example, a high level of activity-induced stress would be associated with a higher threshold for detecting a light-induced discomfort, based on the global level of activity conducted by the user.

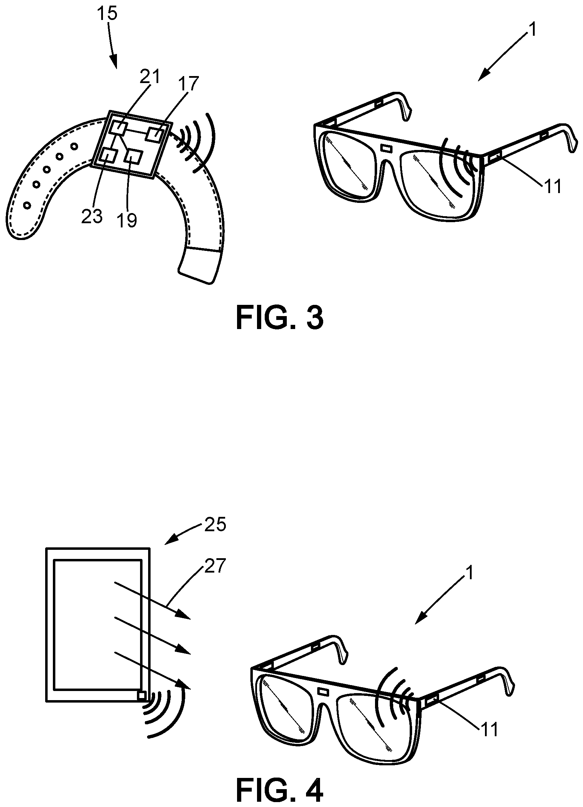

In a second embodiment shown on figure 3, the system 3 comprises several components mounted on an external device 15, in this case a wristband or smart watch.

The external device 15 is for example worn by the user of the system 3, and comprises a communication module 17, configured to communicate with the calculation module 5 through the communication module 11 of the head-mounted device 1.

The external device 15 may be configured to deliver the notifications issued by the notification module 6 to the user, for example through an interface mounted on the external device 15.

The external device 15 may also comprise a sensor 19 for measuring the skin conductance of the user in a second skin region, for example on the wrist. The sensor 19 may supplement or replace the sensor 9 mounted on the head-mounted device 1.

The external device further comprises a control module 21 configured to control the communication module 17 and the sensor 19 mounted on the external device 15.

The system 3 may comprise at least one physiological sensor 23, configured to measure values of at least one physiological parameter of the user. The physiological sensor 15 may be mounted on the head-mounted device 1 or on the external device 15 worn by the user, and in communication with the calculation module 5 through the communication device 11, as shown on figure 3.

The at least one physiological sensor 23 may comprise a thermometer for measuring a skin temperature of the user as the physiological parameter, a heart rate monitor and/or a blood pressure monitor to measure a heart rate and/or blood pressure of the user as the physiological parameter, and/or an electroencephalograph, to measure an electric activity level in the user's brain region as the physiological parameter.

The at least one physiological sensor 23 may also comprise an eye-tracking camera configured to acquire images of the eyes of the wearer, the calculation module 3 being further configured to determine an eye squinting frequency of the user based on the acquired images.

The system 3 is further configured to analyse the measured values of the at least one physiological parameter and determine a physiological stress level of the user based on the values of the at least one physiological parameter and adjust the reference values based on the determined physiological stress level.

For example, a high level of physiological stress would lead to an increased reference threshold required to detect a light-induced stress, to compensate for independent stressing factors.

The system 3 may also be configured to communicate with a luminotherapy or phototherapy device 25 is use by the user, as shown on figure 4, and issue commands to the luminotherapy device 25 to adjust the settings of the luminotherapy device to reduce a detected light-induced discomfort of the user.

For example, a light intensity and/or a color spectrum of a light flux 27 emitted by the luminotherapy device 25 and received by the user may be adjusted to reduce discomfort and be able to maintain the illumination provided by the luminotherapy device 25 as high as possible while staying under the light-induced discomfort threshold. This is particularly beneficial for phototherapy or luminotherapy treatments requiring or benefiting from a prolonged exposure.

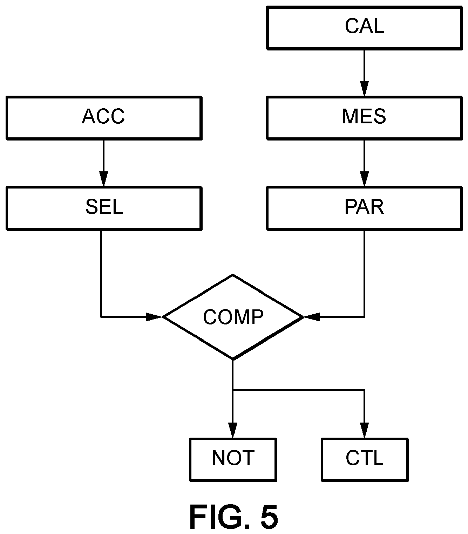

A computer-implemented evaluation method for evaluating a light-induced discomfort of a user of the system 3 will be described below, in reference to figure 5.

The evaluation method may comprise an initial calibration step CAL, during which the reference values are determined specifically for the user of the system 3.

The calibration step may be triggered manually or automatically, for example upon turning on the system 3.

During the calibration step, the system 3 collects values of the at least one parameter representative of the measured skin conductance through the at least one sensor 9, as well as other values obtained by other types of sensors present in the system 3, such as ambient light level values through the light sensor 13, for example.

The system 3 may also collect an input from the user, for example for setting a threshold corresponding to a discomfort experienced by the user.

The evaluation method comprises a step MES of obtaining through the sensor 9 measurements of the electrical conductance of the skin surface of the user.

The measurements of the electrical conductance may be obtained continuously, periodically, of during specific predetermined recoding phases. The measurements may also be triggered by a predetermined external event, such as a minimum threshold of ambient light being reached, and/or a user command.

The measured values of the electrical conductance are stored in the memory 5b of the calculation unit 5 for treatment.

The evaluation method further comprises a step of obtaining PAR at least one value of at least one parameter representative of the measured electrical conductance.

The parameter may be, for example, a frequency of spikes of electrical conductance, an intensity of spikes of electrical conductance, an/or a number of spikes of electrical conductance. In this case, a spike designates a sharp rise of electrical conductance from a current base value, followed by a quick exponential decrease to return to the base value.

During this step, the values of measured conductance stored in the memory 5b are treated by the calculation module 5 with at least one data treatment tools to obtain values of the at least one parameter.

The numerical treatment of the data may comprise a correction of artefacts in the measurements, for example artefacts resulting from a movement of the user. The detection of artefacts is based on the shape of the associated peaks of electrical conductance, with dynamic differing from the above-described spikes.

The numerical treatment may also comprise a smoothing of the curve, for example through a Hann windowing algorithm.

The numerical treatment may also comprise a continuous decomposition analysis, carried out to separate a phasic component from a tonic component of the electrical conductance. The phasic component may comprise, for example, slow variations of the base level, while the tonic component comprises sharp variations, including the spikes used to obtain the values of the parameter.

The numerical treatment may comprise a detection of the spikes by applying a predetermined threshold value to detect if a variation can be considered a spike. The threshold is for example taken as a minimum amplitude variation of 0,05 µS.

The evaluation method may comprise a step ACC of obtaining values of at least one other parameter through measurements form at least one other sensor of the system 3.

For example, values of the level of ambient light may be obtained through measurements from the light-sensor 13, values of the at least one physiological parameter may be obtained through measurements from the physiological sensor 15, and/or values of the at least one activity parameter through measurements from the position and/or movement sensor 14.

The evaluation method may also comprise a step SEL of selecting at least one predetermined reference value for the at least one parameter. The selected reference values are determined based on preregistered user preferences, eventual commands from the user, and, optionally, the obtained values of the other parameters.

The reference values may be further adjusted to compensate for an external source of stress that does not correspond to a light-induced discomfort, based on the values of the other parameters obtained in the step ACC.

The evaluation method also comprises a step COMP of comparing the at least one value of the at least one parameter to the at least one reference value and detecting a light-induced discomfort of the user based on the comparison.

For example, the references values comprise at least one threshold for detecting a light-induced discomfort of the user.

In case of a detected discomfort in the user, the evaluation method may comprise a step NOT of notifying the user of the discomfort, along with at least one optional suggestion to address the discomfort.

The step NOT of notifying may also comprise the registering of data relative to the detected discomfort in the memory 5b, and the registration and/or notification of data for an eye-care practician.

The evaluation method may also comprise a step CTL of controlling an optical element 7 to address the discomfort, or to control an external device such as a luminotherapy device to address the discomfort.

The method may either comprise the step NOT of notifying the user and/or the step CTL of controlling as described above.

## Claims

1. A system (3) for evaluating a light-induced discomfort of a user, the system (3) comprising a sensor (9) for measuring an electrical conductance of a skin surface of the user, the system (3) being configured for:
- obtaining through the sensor (9) at least one measurement of the electrical conductance of the skin surface of the user,
- obtaining at least one value of at least one parameter representative of the at least one measurement of the electrical conductance, and
- comparing the at least one value of the at least one parameter to at least one reference value and detecting a light-induced discomfort of the user based on the comparison.

2. The system (3) according to claim 1, wherein the at least one parameter comprises a frequency of spikes of the measured electrical conductance, an amplitude of spikes of the measured electrical conductance, and/or a delay between spikes of the measured electrical conductance.

3. The system (3) according to claim 1 or 2, wherein the system (3) comprises a notification module (6) configured to issue notifications to the user, through an interface and/or through an external electronic device (15), the system (3) being also configured for:
- issuing a notification of a detected light-induced discomfort of the user and, optionally, a suggestion to adjust or replace an optical element (7) worn by the user.

4. The system (3) according to any one of claims 1 to 3, wherein the system (3) comprises a communication module (11) configured to communicate with at least one controllable optical element (7, 8) presenting a variable optical function, worn by the user, the system (3) being also configured for:
- issuing a command to the controllable optical element (7, 8) to modify the variable optical function of the controllable optical element (7, 8) to reduce the detected light-induced discomfort of the user.

5. The system (3) according to claim 4, wherein the controllable optical element (7, 8) is a diming element the command to modify the optical function comprising adjusting a transmission value of the diming element and/or a polarization value of the diming element and/or a transition speed between at least two states of the diming element.

6. The system (3) according to claim 4 or 5, the communication module being a first communication module,
wherein the system (3) comprises a second communication module (11) configured to control a luminotherapy device (25) in use by the user, the system (3) being also configured for:
- issuing a command to the luminotherapy device (25) of adjusting settings of the luminotherapy device (25) to reduce the detected light-induced discomfort of the user.

7. The system according to any one of claims 1 to 3, wherein the system comprises a communication module configured to communicate with a luminotherapy device in use by the user, the system being also configured for:
- issuing a command to the luminotherapy device of adjusting settings of the luminotherapy device to reduce the detected light-induced discomfort of the user.

8. The system (3) according to any one of claims 1 to 7, wherein the sensor (9) is arranged in a frame (2) of a head-mounted device (1) or arranged in an external device (15) worn by the user in contact with the skin surface of the user.

9. The system (3) according to any one of claims 1 to 8, wherein the system (3) further comprises at least one sensor (13) for determining an amount of an ambient light, the system (3) being also configured for:
- comparing the amount of the ambient light with a predetermined ambient light threshold,
- detecting the light-induced discomfort also based on the comparison of the amount of the ambient light with a predetermined ambient light threshold.

10. The system (3) according to any one of claims 1 to 8, wherein the system (3) further comprises at least one position and/or motion sensor (14), the system (3) being also configured for:
- obtaining values of the at least one activity parameter representative of a position and/or a motion of the user through the at least one position and/or motion sensor (14),
- determining an activity-induced stress level based on the values of the at least one activity parameter, and
- adjusting the reference values based on the determined activity-induced stress level.

11. The system (3) according to any one of claims 1 to 10, wherein the system further comprises at least one physiological sensor (23) configured for obtaining values of at least one physiological parameter of the user, the system being also configured for:
- obtaining values of the at least one physiological parameter of the user through the at least one physiological sensor (23),
- determining a physiological stress level based on the values of the at least one physiological parameter, and
- adjusting the reference values based on the determined physiological stress level.

12. The system (3) according to any one of claims 1 to 11, wherein the reference values are determined specifically for the user in a preliminary calibration process (CAL).

13. A head-mounted device (1) comprising a frame (2), at least one optical element (7) and a system (3) for evaluating a light-induced discomfort of a user, the system (3) comprising a sensor (9) for measuring an electrical conductance of a skin surface of the user, the system (3) being configured for:
- obtaining through the sensor (9) measurements of the electrical conductance of the skin surface of the user,
- obtaining at least one value of at least one parameter representative of the measured electrical conductance, and
- comparing the at least one value of the at least one parameter to at least one reference value and detecting a light-induced discomfort of the user based on the comparison.

14. Eyeglasses (1) comprising a frame (2), at least one optical element (7) and a system (3) for evaluating a light-induced discomfort of a user, the system (3) comprising a sensor (9) for measuring an electrical conductance of a skin surface of the user, the system (3) being configured for:
- obtaining through the sensor (9) measurements of the electrical conductance of the skin surface of the user,
- obtaining at least one value of at least one parameter representative of the measured electrical conductance, and
- comparing the at least one value of the at least one parameter to at least one reference value and detecting a light-induced discomfort of the user based on the comparison.

15. A computer-implemented evaluation method for evaluating a light-induced discomfort of a user, the evaluation method comprising:
- obtaining (MES) through the sensor (9) measurements of the electrical conductance of the skin surface of the user,
- obtaining (PAR) at least one value of at least one parameter representative of the measured electrical conductance, and
- comparing (COMP) the at least one value of the at least one parameter to at least one reference value and detecting a light-induced discomfort of the user based on the comparison.
